(19) **Europäisches Patentamt**

**European Patent Office**

**Office européen des brevets**

(11) **EP 0 601 508 B1**

(12) **EUROPEAN PATENT SPECIFICATION**

(45) Date of publication and mention
of the grant of the patent:
**31.03.1999 Bulletin 1999/13**

(51) Int. Cl.$^6$: **A61K 31/19**, A61K 9/50,
A61K 9/52, A61K 9/10,
A61K 9/00

(21) Application number: **93119587.9**

(22) Date of filing: **06.12.1993**

(54) **Liquid-suspension controlled-release pharmaceutical composition**

Pharmazeutische flüssige Suspensionszusammensetzung mit kontrollierter Freigabe

Composition pharmaceutique de suspension liquide à libération contrôlée

(84) Designated Contracting States:
**AT BE CH DE DK ES FR GB GR IE IT LI LU MC NL PT SE**

(30) Priority: **11.12.1992 IT MI922826**

(43) Date of publication of application:
**15.06.1994 Bulletin 1994/24**

(73) Proprietor:
**RECORDATI S.A.**
**CHEMICAL and PHARMACEUTICAL COMPANY**
**6830 Chiasso (CH)**

(72) Inventors:
• **Santus, Giancarlo**
  **I-20146 Milan (IT)**
• **Bottoni, Giuseppe**
  **I-24100 Bergamo (IT)**
• **Bilato, Ettore**
  **I-35121 Padova (IT)**

(74) Representative:
**Gervasi, Gemma, Dr.**
**NOTARBARTOLO & GERVASI Srl,**
**Corso di Porta Vittoria, 9**
**20122 Milano (IT)**

(56) References cited:
| | |
|---|---|
| **EP-A- 0 308 739** | **EP-A- 0 313 328** |
| **EP-A- 0 359 195** | **EP-A- 0 413 533** |
| **EP-A- 0 438 249** | **WO-A-85/04099** |
| **CH-A- 643 455** | **GB-A- 2 166 651** |

EP 0 601 508 B1

## Description

### Field of the invention

The invention relates to controlled-release pharmaceutical formulations in liquid dosage forms for the administration of naproxen.

### Background of the invention

(S)-6-methoxy-$\alpha$-methyl-2-naphtaleneacetic acid (naproxen) is a long-known non-steroid anti-inflammatory drug, which also has analgesic and antipyretic activity (patents U.S. 3,904,682 and U.S. 4,009,197).

Because of its anti-inflammatory and analgesic activities, it is indicated for treating various forms of arthritis, such as rheumatoid arthritis, osteoarthritis, ankylosing spondylitis, and gout arthropathy; as well as for treating forms of extraarticular rheumatism, such as lumbar sciatica, myalgia, neuralgia, radicular syndromes, periarthrites, myofibrosites and the like.

Therapy generally involves the use of daily doses ranging from 250 mg to 1,000 mg of active ingredient and the formulations currently available for administration include tablets, capsules, granules for extempore suspension (oral route), suppositories (rectal route), vials (intramuscular route), gels and emulsions (topical route). All these formulations are essentially designed to make the active ingredient readily available in the body in order to ensure a rapid onset of the therapeutic effect.

In particular, with respect to the oral administration route, the wish to extend this therapeutic effect in time led to the development of controlled drug release formulations in order to have one single daily administration. It is in fact known that once-daily administration enables patients to better comply with recommended dosage during the therapeutic treatment. For instance, the "patient compliance" rate in the use of drugs is reported to vary from 87% for drugs to be taken once a day to 39% for drugs to be taken four times a day [J. A. Cramer et al., *JAMA* It. ed. 1, 601 (1989)].

Examples of formulations suitable for once-daily administration are reported in US Patent 4,803,079, which describes the use of a matrix in which the active ingredient is dispersed and released by diffusion, as well as in European Patent Application EP 458,249 which describes the use of slow release pellets for the administration of a delayed release dose together with a ready release dose; and in European Patent EP 250,374, which describes solid dosing units suitable for the controlled release of drugs, including naproxen, with zero order kinetics.

The main drawback of all these applications is the final size of the solid dosage unit which, particularly for dosages with a high active ingredient content, as is the case with naproxen, leads to the development of dosage forms with high weights and large sizes. Thus, for instance, by applying the teachings of patent US 4,803,079, a tablet containing 1,000 mg of active ingredient would have an overall weight of about 1,300 mg, a diameter of about 15 mm, and a thickness of about 7 mm.

Obviously, a dosage unit with these characteristics involves considerable administration problems in patients with deglutition difficulty, and this is not a minor problem considering that, for the above types of diseases, the patient population that uses naproxen is generally formed by elderly individuals.

Even when the systems involve breaking down the formulation into micro-units (granules or pellets), the need to avoid overdose phenomena, which in the case of naproxen may induce torpor, gastric pyrosis, dyspepsia, nausea or vomiting, requires that the micro-units be in any case vehiculated by using a container (e.g. a gelatine capsule) that will consequently have dimensions similar to the ones above, and, therefore, deglutition problems will remain unsolved.

A further disadvantage of the oral administration of solid controlled-release dosage forms in the case of naproxen is that the active ingredient either as a tablet or as a capsule may give rise to problems of local gastrointestinal contact tolerance which may occasionally cause bleeding in the gastrointestinal tract, peptic ulcer or colitis.

A solution to the problems caused by solid controlled-release dosage forms may be the use of liquid-suspension controlled-release formulations. Because of the intrinsic characteristics of these formulations, they are required to be particularly homogeneous, suspendable and palatable as well in a form that guarantees the required release profile, and this implying that the controlled release suspensions be formed by particles of sizes allowing compliance with the above requirements.

Generally, the requirement for these small dimensions means keeping a low excipient quantity ratio in order not to affect suspension and to avoid a "sand-like" sensation upon ingestion. It is also particularly necessary to maintain high active ingredient contents in the microgranules when the treatment conditions require a high dosage, as in the case of naproxen.

For instance, in the formulation described in the international patent application WO 89/8448, the enteric-coated granules have in any case diameters larger than 0.5 mm and, therefore, are totally unsuitable for suspension in liquid formulations.

Examples of formulations dimensionally suitable for liquid suspension for the administration of naproxen are described in the Belgian patent BE 903,540, disclosing a powder controlled-release composition consisting of particles of sizes ranging from 0.1 to 125 μm which can be used for the liquid administration of several drugs, including naproxen.

Also EP 359,195 describes a therapeutic system for liquid controlled-release pharmaceutical compositions that can be used to administer naproxen. This system consists of microgranules coated first with a pH-insensitive (inner) coating that imparts controlled release property to the microgranules followed by various alternate coatings of lipophilic and hydrophilic materials of the total size of the microgranules ranges from 50 to 500 μm. The microgranules form stable suspensions in liquid administration vehicles.

However, by applying the teachings of the foregoing patents and applications, no liquid suspension formulations can be developed being capable of achieving therapeutically effective naproxen levels by a single daily administration and, furthermore, none of the liquid formulations described in the above patent applications is designed so as to pass through the gastric fluid barrier. In addition, the above mentioned EP 359,195 Application does not describe any specific working examples where naproxen is the active ingredient and does not specifically disclose a liquid controlled release formulation for naproxen suitable for a once-a-day administration nor a naproxen containing liquid formulation capable of withstanding gastric fluid.

The need was felt for a controlled-release pharmaceutical composition, in liquid suspension, capable of ensuring therapeutically active naproxen blood levels with only one daily administration, designed so as to avoid the detrimental effects that may be caused by a prolonged contact of the active ingredient with the gastric mucous membrane.

## Summary of the invention

It has now surprisingly been found that, if the granules containing naproxen - obtained in accordance with what is described in patent application EP 359,195 - are coated with a series of four coatings in such a way that the first coating applied to the granules always contains polyethylene glycol and, at the same time, the outermost coating always contains cellulose acetate phthalate (CAP) having the property of forming enteric coatings with a plasticizer, a formulation capable of solving the above technical problems is obtained.

## Detailed description of the invention

This formulation is thus capable of preserving the release characteristics of the dosage forms therein contained and can be designed either as a liquid dosage formulation that would remain stable for a relatively long period of time, or as a liquid formulation that is prepared when needed and will then remain stable throughout the period in which the treatment is carried out.

Since a significant improvement of patient compliance can be achieved by the present invention, its advantages in terms of a reduction of the number of daily doses while retaining intact the required ease of administration and deglutition are clear. Additionally, an improved therapeutic response can be expected as dosage can be adjusted to requirements simply by measuring the necessary suspension volumes. Finally, the outermost coating enhances local contact tolerance resulting in a reduction of undesirable side effects.

The concentration of naproxen reaching a gut mucosal cell depends both upon the drug concentration in the lumen of the gut and the plasma concentration of the drug in mucosal capillaries.

It is generally believed that the mucus barrier in vulnerable patients is somewhat deficient. Naproxen penetrates this barrier, inhibits the formation of the prostaglandins that sustain the mucus barrier and thereby allows hydrogen ions coming from gastric hydrochloric acid to leak back and damage the mucosa.

This leads towards the possibility of a drug formulation which would avoid the problems of dispersion and topical contact with the gastric and upper duodenal mucosa. An enteric coating that is stable at low pH and dissolves at a higher (duodenal) one offers this option. Thus it would be reasonable to expect comparable efficacy and improved upper gastrointestinal tract safety for the enteric formulation.

In detail, the invention is a liquid measurable enteric-coated controlled-release pharmaceutical composition comprising:

1) Dosage forms for the controlled release of naproxen having sizes ranging between 50 and 500 μm (preferably 90 and 300 μm), suitable to remain easily in suspension in a liquid for prolonged periods of time, each of said forms consisting essentially of:

a) a mixture of naproxen and excipients treated so as to form a microgranular core of substantially uniform spherical surface free from sharp or discontinuous morphology and permitting depositions of uniform coating thereon to ensure reproducibility and uniform distribution of the successive coatings;

b) a first coating in contact with said microgranular core to form a barrier unaffected by pH variations and imparting controlled release properties to the naproxen contained in the core;

c) a second coating having essentially hydrophylic properties, superimposed onto the first one;
d) a third lipophilic coating on the second one;
e) a final outer coating with "enteric" characteristics, meaning that it is formed by substances resisting to the dissolution in gastric fluids, but disintegrating in the small intestine.

2) A vehicle for the above controlled-release forms consisting of one of the following alternatives:

a) a dry mixture of suspending agents, sweetening agents and the controlled-release forms described in 1), capable of achieving a formulation to be reconstituted when needed;
b) an aqueous solution of the above suspending and sweetening agents in which the controlled release forms described in 1) can be suspended and maintained in optimum release conditions for extended periods of time.

In accordance with the present invention it has then been found that granules with a high content of active ingredient (80-90% by weight), having sizes after coating within the range 50-500 $\mu$m (preferably 90-300 $\mu$m), uniform surfaces, preferably having almost spherical shapes, apparent densities ranging from 300 to 800 g/l (preferably 500-600 g/l) and very low friabilities, can be obtained by wet mixing naproxen and excipients by known techniques described in EP 359,195.

The elements characterizing the ingredients of the pharmaceutical formulation of the present invention are described hereinbelow:

Microgranular core - 1 a):

The excipients used to prepare the core can be selected from those commonly used in wet mixing, such as dibasic calcium phosphate, lactose, microcrystalline cellulose, starch, talc, sugars, polyvinylpyrrolidone, polyvinylpyrrolidone/vinyl acetate copolymer and the like. The mixing liquid can be water or a water soluble solvent such as ethyl alcohol or other commonly used alcohols, or a mixture of water and alcohol.

Again according to the disclosed invention, the granulate is then coated in successive stages with coatings of different compositions, using known coating techniques. As an example, the materials forming the three different types of coating used are reported hereinbelow:

Coating that controls release - 1 b):

Mixture of a first ingredient which is a cellulose derivative, such as ethyl cellulose, with a second plasticizing ingredient, such as diethyl phthalate in the presence of polyethylene glycol.

It has, in fact, been found that the addition of 0.1-0.5% by weight on the first coating weight polyethylene glycol to the coating mixture gives the membrane responsible for controlling release, in addition to the known plastic characteristics, hydrophilic properties which are useful for the diffusion of substances not readily soluble in water such as naproxen.

Hydrophilic coating - 1 c):

Hydrophilic substances such as methacrylic acid, methyl cellulose or polyvinylpyrrolidone.

Lipophilic coating - 1 d):

Fatty substances such as mono-, di- or triglycerides of fatty acids having from 6 to 32 carbon atoms waxes such as carnauba wax, beeswax, candelilla wax, fatty alcohols and fatty acids.

Enteric Coating - 1 e):

Hydrophilic substances such as cellulose acetate phthalate, hydroxypropyl methyl cellulose phthalate, cellulose acetate trimellitate. The same substances forming the enteric coating 1 e) may also be used as ingredients for the second hydrophylic coating 1 c).

Plasticizers are preferably added to type 1 c) and 1 e) coating materials. The choice of a plasticizer for these coatings depends on whether it will be used for a wet process or for a process using organic solvents. Chlorinated solvents such as chloroform, alcohols such as ethanol, methanol or isopropyl alcohol, ketones such as acetone or methyl ethyl ketone, and different mixtures of the above solvents can in fact be used as coating solvents as well as water.

Among the various usable plasticizers, such as for instance diethyl phthalate, dibutyl sebacate, triacetin, trialkylcitrate, vegetable oils, acetylated glycerides, polyethylene glycols or propylene glycols, the one which was found to be most suitable for the composition of the invention is diethyl phthalate.

Diethylphthalate is preferably used in the polymeric coatings in a quantity ranging from 10 to 30% by weight based on the weight of the specific (first, second or fourth) coating.

Vehicle - 2 a) and 2 b):

The granulate coated according to the above procedures can then be combined with a vehicle either as a solid mixture which can be suspended *extempore* when needed - 2 a), or into a suspension ready for use - 2 b).

In addition to the quantity of coated microgranules containing doses of the controlled-release active ingredient, the ingredients forming the vehicle are:

- suspending and thickening agents such as cellulose esters, microcrystalline cellulose, alginic acid derivatives, polyvinylpyrrolidone derivatives;
- sugars such as sucrose and sorbitol;
- buffering agents such as citric acid and sodium citrate, glycine and hydrochloric acid, sodium and potassium phosphates;
- preservatives and bacteriostatic agents such as p-hydroxybenzoic acid esters;
- various flavorings and sweeteners commonly used in pharmaceuticals.

In addition to the above ingredients, the dosage form to be combined with a vehicle as ready for use also comprises water or mixtures of water and co-solvents such as glycol, alcohol, and glycerin. The methods, tables and examples reported hereinbelow are meant to better describe the subject invention and demonstrate its advantages and applicability.

## EXAMPLE 1

Preparation of the Microgranulate

A mixture formed by 3,400 g naproxen, 400 g polyvinylpyrrolidone and 400 g lactose is mixed for 5 minutes so as to ensure good homogeneity. 500 ml of water atomized at a 2 bar pressure are then added to the mixture under stirring at a flow rate of 35 ml/min. The granulate is then made spheroidal by stirring the mixture for an additional 10 minutes. The spheroidal product is then dried for about 2 hours on a temperature-controlled static bed at 35°C until the residual humidity is reduced to a level of 4-5% by weight. The spheroidal product is sieved through 0.6 mm sieves until a granulate with a size range from 90 to 300 $\mu$m and a spheroidal shape with no particular surface roughness or discontinuity is obtained. The granulate portions with sizes smaller than 90 $\mu$m or larger than 300 $\mu$m are separated, pulverized in a mill and re-used in further production cycles.

The microgranulate is characterized for particle size distribution and density. These characteristics are necessary to calculate the surface area of the microgranulate.

By mathematical processing of the particle size distribution data, $d_g$ (mean geometric diameter) and $\sigma_g$ (standard deviation) of particle size distribution are calculated. Then $d_{vs}$ is calculated as follows from $d_g$ and $\sigma_g$. $\log d_{vs} = \log d_g - 1.151 \log^2 \sigma_g$ , wherein $d_{vs}$ is the diameter volume-surface.

The surface area is evaluated by means of the following formula: surface area = $6 / P_g * d_{vs}$ wherein $P_g$ is the apparent density.

Taking into account the surface area value then it is possible to apply a constant amount of coating on the microgranules.

## EXAMPLE 2

500 ml of atomized water at a 15 ml/min flow rate and a 2 bar pressure are added to a mixture formed by 3,560 g naproxen and 440 g polyvinylpyrrolidone mixed as described in Example 1. The granulate is then rendered spheroidal, dried and sieved as previously described.

## EXAMPLE 3

450 ml of atomized water at a 20 ml/min flow rate and a 2 bar pressure are added to a mixture formed by 3,200 g naproxen, 400 g polyvinylpyrrolidone and 400 g dibasic calcium phosphate dihydrate mixed as described in Example 1. The granulate is then rendered spheroidal, dried and sieved as previously described.

### EXAMPLE 4

500 ml of atomized water at a 15 ml/min flow rate and a 2 bar pressure are added to a mixture formed by 3,200 g naproxen, 400 g polyvinylpyrrolidone, 200 g lactose and 200 g dibasic calcium phosphate dihydrate and mixed as described in Example 1. The granulate is then rendered spheroidal, dried and sieved as previously described.

### EXAMPLE 5

Coating of Microgranulate

First Coating in Contact with the Core

500 g of microgranulate prepared in accordance with the process of Example 1 are introduced into a fluid-bed coating apparatus, into which air heated to a temperature of 40-45°C is blown at a rate of 40-45 $M^3$/hour, under turbulence stirred for 1 minute. A solution having the composition percent by weight hereinbelow reported is sprayed at a 2 bar pressure and at 16 g/min flow rate onto the microgranulate:

| | |
|---|---|
| Ethyl cellulose | 3.00 |
| Diethyl phthalate | 1.00 |
| Polyethylene glycol | 0.10 |
| Ethyl alcohol | 21.35 |
| Chloroform | 74.55 |

The quantity of material used for coating is in relation to the surface area to be coated. For example: 585 g of the above solution are sprayed to coat a surface area of 1.6 g /m $^2$, whereas 731 g of the same are necessary for coating 2.0 g/m $^2$.

### EXAMPLE 6

Alternatively, following the operating conditions as described in Example 5, the microgranulate may be coated with a solution having the following percent composition by weight:

| | |
|---|---|
| Ethyl cellulose | 2.5 |
| Diethyl phthalate | 1.0 |
| Polyethylene glycol | 0.2 |
| Ethyl alcohol | 96.3 |

### EXAMPLE 7

Following the operating conditions as described in Example 5, the microgranulate may be coated with a solution having the following percent composition by weight:

| | |
|---|---|
| Ethyl cellulose | 3.0 |
| Diethyl phthalate | 1.0 |
| Ethyl alcohol | 21.0 |

(continued)

| | |
|---|---|
| Chloroform | 75.0 |

## EXAMPLE 8

Second Coating (Hydrophilic Coating)

The same operating conditions described as in Example 5 for applying the first coating is also used to apply the hydrophilic coating. A solution having the following percent composition are applied at a 2 bar pressure and an 8-10 g/min flow rate:

Methacrylic acid copolymer

| | |
|---|---|
| Eudragit E [R] | 12.5 |
| Acetone | 35.0 |
| Isopropyl alcohol | 52.5 |

The amount of solution used for the second coating is in relationship with the amount used for the first coating. For example: 50 g of the above solution are used when 1.6 g /$m^2$ of the first coating is applied, whereas 62.4 g of the same are necessary when 2.0 g/$m^2$ is used.

## EXAMPLE 9

Alternatively, the first coating may be coated with a solution having the following percent composition by weight:

| | |
|---|---|
| Methylcellulose | 3.0 |
| Water | 97.0 |

## EXAMPLE 10

Another alternative solution suitable for the second coating layer is (percent composition by weight):

| | |
|---|---|
| Polyvinylpyrrolidone | 5.0 |
| Water | 95.0 |

## EXAMPLE 11

Third Coating (Lipophilic Coating)

A solution having the following percent composition is applied to the second coating at the same pressure and flow conditions described in Example 5 for the first coating:

| Glyceryl monostearate | 4.50 |
|---|---|
| White beeswax | 0.40 |
| Cetyl alcohol | 0.05 |
| Stearyl alcohol | 0.05 |
| Chloroform | 89.60 |
| Methanol | 5.40 |

The amount of solution used for the third coating is in relationship with the amount used for the first coating.

For example: 577 g of the above solution are used when 1.6 g /m$^2$ of the first coating is applied, whereas 697 g of the same are necessary when 2.0 g/m$^2$ is used.

**EXAMPLE 12**

Alternatively, the second coating may be coated with a solution having the following percent composition by weight:

| Glyceryl monostearate | 6.5 |
|---|---|
| Chloroform | 93.5 |

**EXAMPLE 13**

Another alternative solution suitable for the third coating layer is (percent composition by weight):

| Beeswax | 6.0 |
|---|---|
| Isopropyl alcohol | 5.0 |
| Chloroform | 89.0 |

**EXAMPLE 14**

A further alternative solution suitable for the third coating layer is (percent composition by weight)

| Kaomel$^R$ | 6.0 |
|---|---|
| Methyl alcohol | 29.0 |
| Chloroform | 65.0 |

Where Kaomel$^R$ is a mixture of hydrogenated vegetable oils of nonlauric origin.

**EXAMPLE 15**

Or in alternative (percent composition by weight):

| Carnauba wax | 5.0 |
|---|---|
| Chloroform | 95.0 |

## EXAMPLE 16

Fourth Coating (Enteric Coating)

The same operating conditions as described in Example 8 for applying the second coating are used for the final enteric coating (percent composition by weight).

| Cellulose acetate phthalate | 4.0 |
|---|---|
| Diethyl phthalate | 1.0 |
| Acetone | 71.2 |
| Isopropyl alcohol | 23.8 |

The amount of solution used for the fourth coating is in relationship with the amount used for the first coating.
For example: 155 g of the above solution are used when 1.6 g /m$^2$ of the first coating is applyed, whereas 196 g of the same are necessary when 2.0 g/m $^2$ is used.

## EXAMPLE 17

Alternatively, the third coating may be coated with a solution having the following percent composition by weight:

| Cellulose acetate trimellitate | 4.0 |
|---|---|
| Diethyl phthalate | 1.5 |
| Acetone | 70.0 |
| Isopropyl alcohol | 24.5 |

## EXAMPLE 18

Another alternative of solution suitable for the final coating layer is (percent composition by weight):

| Hydroxypropylmethylcellulose phthalate | 4.0 |
|---|---|
| Diethyl phthalate | 1.0 |
| Acetone | 75.0 |
| Isopropyl alcohol | 21.0 |

## EXAMPLE 19

Preparation of Controlled-Release Liquid Suspension

5.50 g microcrystalline cellulose, 0.50 g sodium carboxymethyl cellulose, 0.50 g sodium citrate, 0.75 g citric acid monohydrate, 0.25 g methyl p-hydroxybenzoate, 0.06 g propyl p-hydroxybenzoate, 0.05 g sodium chloride, 0.02 g glycyrrhizinated ammonium and 65.77 g sugar are mixed in a mixer-granulator after sieving through a 0.4 mm average mesh. After dispersing 0.05 g surfactant (Span 20) and 0.20 g antifoam (dimethylpolysiloxane) in 5 ml of water, the resulting liquid is mixed with the powder mixture and granulated in the mixer-granulator. At the end, after drying to constant humidity, the resulting granulate is mixed with 1.30 g tragacanth and 0.05 g powdered citrus flavoring.

25.0 g of controlled-release naproxen microgranules, obtained as described in Examples 1, 5, 8, 11, 16, are finally mixed with the vehicle granulate so as to obtain 100 g of formulation ready to be suspended in sufficient water to yield a naproxen content of 750 mg in 10 ml of final suspension.

## EXAMPLE 20

In Vitro Release Testing

Release is tested using apparatus II (paddle) in the United States Pharmacopoeia Ed. XXI operating at 50 revolutions/min in 900 ml of a phosphate buffer (pH = 7.4) having the following composition:

| | | |
|---|---|---|
| Dibasic sodium phosphate dihydrate g | | 14.40 |
| Monobasic sodium phosphate dihydrate g | | 2.96 |
| Demineralized water          q.s. to ml | | 1,000. |

The active ingredient concentration is determined by a spectrophotometric technique or by high performance liquid chromatography with a U.V. detector at 332nm.

Table 1 shows the naproxen percentages released by microgranules that differ from each other only with respect to the first coatings which are prepared as per Examples 5 and 7 respectively. It is clear that, conditions being equal, the use of polyethylene glycol as one of the ingredients of the 1st coating (Example 5) ensures an optimum control over the release of naproxen over 24 hours.

TABLE 1

| Time (Hours) | % Naproxen Released | |
|---|---|---|
| | Example 5 | Example 7 |
| 1 | 41 | 15 |
| 2 | 55 | 21 |
| 4 | 67 | 30 |
| 8 | 79 | 44 |
| 12 | 85 | 53 |
| 24 | > 90 | 65 |

## EXAMPLE 21

Testing of Resistance to Gastric Fluids

Testing of resistance to gastric fluids is performed using the six vessels apparatus described in the United States Pharmacopoeia Ed. XXII page 1580. Tests are carried out at 37°C and 60 revolutions/minute in 750 ml 0.1 N hydrochloric acid. Samples are taken after the first and second treatment hours. Coated microgranules prepared and coated as described in Examples 1, 5, 8, 11 and 16 are used in comparison with microgranules coated with a first layer of ethylcellulose without a superimposed enteric coating.

The individual measured percentages of dissolved naproxen are reported in Table 2.

A comparison between the data obtained from the full coated microgranules and the data from the microgranules partially coated shows how in none of the first six samples tested the drug fractions dissolved in the medium exceed 10% of the doses introduced, therefore the tested microgranules meet the first USP XXII enteric-coating specification.

On the contrary, the six samples lacking of the enteric coating are out of the USP requirements even after the first hour.

TABLE 2

| Samples | % Naproxen Dissolved | | |
|---|---|---|---|
| | Full coated | | 1st coat. |
| 1st Hour | 2nd Hour | 1st Hour | |
| 1 | 1.3 | 2.1 | 16.0 |
| 2 | 1.0 | 2.0 | 18.0 |
| 3 | 1.6 | 1.9 | 18.0 |
| 4 | 1.3 | 2.0 | 17.5 |
| 5 | 1.4 | 1.8 | 16.0 |
| 6 | 1.3 | 1.9 | 19.0 |

## EXAMPLE 22

### Testing of Dissolution Stability in Time

Tables 3 and 4 show the stability at room temperature of two formulations containing microgranules prepared and coated as described in Examples 1, 5, 8, 11 and 16 and differing from each other only in the quantity of material used for coating in relation to the surface area to be coated:

Table 3 = 1.6 g/m$^2$, Table 4 = 2.0 g/m$^2$

The release of naproxen is measured by a UV technique, using the dissolution method described in Example 20, at the initial time and after 30 days.

TABLE 3

| (1.6 g/m$^2$) | | |
|---|---|---|
| Time (Hours) | % Naproxen Release | |
| | Initial | 30 Days |
| 1 | 41 | 42 |
| 2 | 55 | 55 |
| 4 | 67 | 68 |
| 8 | 79 | 80 |
| 12 | 85 | 85 |
| 24 | > 90 | > 90 |

TABLE 4

| (2.0 g/m$^2$) | | |
|---|---|---|
| Time (Hours) | % Naproxen Release | |
| | Initial | 30 Days |
| 1 | 20 | 22 |
| 2 | 27 | 30 |
| 4 | 38 | 42 |
| 8 | 52 | 55 |
| 12 | 59 | 60 |
| 24 | > 80 | > 80 |

It is clear that in both cases the product retains unchanged release characteristics even after one month, and that the larger quantity of coating applied (Table 4) causes a reduction of the active ingredient release.

## EXAMPLE 23

Testing of Suspendability in Vehicle

Suspendability in the vehicle was tested by pouring the suspension described in Example 19, after stirring it for about 30 seconds, into a graduated cylinder and measuring the height of the clear liquid against total liquid height at fixed times. After one hour all particles were still in suspension. After 24 hours the height of the clear liquid was only 5% of the total. The present suspension formulation ensures consistent and homogeneous contents of any different doses taken.

## EXAMPLE 24

Bioavailability and Therapeutic Efficacy of Formulation

A kinetic study was carried out according to a cross-over design in two periods using single doses, to test the bioavailability and therapeutic efficacy of the invention.
The study was conducted in 6 healthy volunteers who received a single dose (750 mg) of a liquid controlled-release (c.r.) naproxen formulation (A) prepared as described in Example 19 and, having the following composition:

| Ingredient | mg |
|---|---|
| Naproxen c.r. microgranules | 25.00 |
| Citric acid | 0.75 |
| Sodium citrate | 0.50 |
| Microcrystalline cellulose | 5.50 |
| Sodium carboxymethylcellulose | 0.50 |
| Tragacanth gum | 1.30 |
| Methyl p-hydroxybenzoate | 0.25 |
| Propyl p-hydroxybenzoate | 0.06 |
| Sorbitan monolaurate | 0.05 |
| Dimethylpolysiloxane | 0.20 |

(continued)

| Ingredient | mg |
|---|---|
| Powdered citrus flavoring | 0.05 |
| Glycyrrhizinated ammonium | 0.02 |
| Sodium chloride | 0.05 |
| Sugar | 65.77 |

As a reference, a package of immediate-release granular naproxen (B) having the following composition was used:

| Ingredient | mg |
|---|---|
| Naproxen | 750.0 |
| Mannitol | 750.0 |
| Polyvinylpyrrolidone | 75.0 |
| Methacrylic acid copolymer | 112.5 |
| Sodium saccharin | 52.5 |
| Lemon flavoring | 150.0 |
| Citric acid | 130.5 |
| Silica | 7.5 |
| Sucrose | 2,467.5 |

Blood samples were taken at different times and the naproxen plasma concentration was determined by high performance liquid chromatography. Table 5 shows the main pharmacokinetic parameters resulting from the trial. These data show that formulation (A) reduced $C_{max}$ considerably and extended $T_{max}$, indicating a relative bioavailability (F) equal to 81% as compared with the reference product (B).

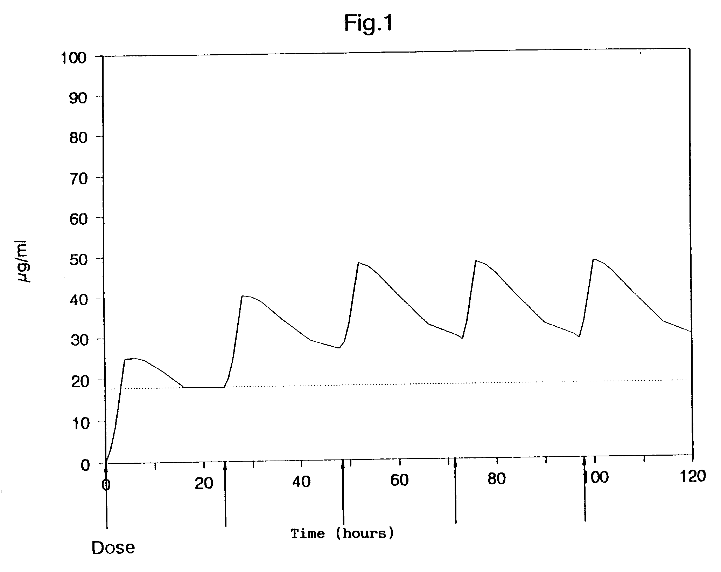

A literature value of 18 μg/ml, which is considered the minimum plasma level ensuring therapeutic efficacy [Clin. Pharm. Ther. 31, 6 (1982)], was used as a reference value to assess therapeutic efficacy. Figure 1 is a graph showing simulated steady-state blood levels after multiple administrations for 5 days, calculated in accordance with the pharmacokinetic parameters found. As can be seen from the figure, the present invention is capable of maintaining therapeutic levels even with a once-daily administration avoiding the initial peak effect and, therefore, minimizing any occurrence of side effects associated with it.

TABLE 5

| PHARMACOKINETIC PARAMETERS (mean values ± S.D.) | | | |
|---|---|---|---|
| | $C_{max}$ (μg.h/ml) | $T_{max}$ (h) | $AUC_{o-\infty}$ (μg/ml) |
| NAPROXEN (A) | 27.12 (± 11.3) | 6.0 (± 1.8) | 946.51 (± 311.21) |
| NAPROXEN (B) | 76.82 (± 18.53) | 2.3 (± 0.8) | 1,168.58 (± 285.63) |

Wherein:
S.D. = Standard deviation
$C_{max}$ = (Peak concentration) is the highest plasma concentration the drug reaches after the administration;
$T_{max}$ = (Time concentration) is the time necessary to reach the $C_{max}$ value;
$AUC_{o-\infty}$ = (area under the curve) is the total area of the time-concentration profile and represents a measure of the bioavailability.

The relative bioavailability (F) of two different forms (A and B) administered at the same dosage and using the same administration route is given by the following formula:

$$F \text{ (relative bioavailability)} = AUC(A)/AUC(B) * 100$$

## Claims

1.  A controlled-release pharmaceutical composition suitable for a once-daily administration of naproxen in a liquid suspension, comprising:

    -   a multiplicity of microgranules containing naproxen as an active ingredient and at least one excipient, of sizes ranging from 50 to 500 $\mu$m, said microgranules having substantially no controlled-release property prior to coating,
    -   a series of four successive polymeric layers, coating said microgranules, this series consisting of:
    -   a first coating applied directly to the microgranules, imparting controlled-release properties to said microgranules according to a predetermined release profile,
    -   a second applied coating with hydrophilic characteristics
    -   a third applied coating with lipophilic characteristics, and
    -   a fourth applied coating with enteric-coating characteristics;

    a liquid administration vehicle for the above coated microgranules characterized in that the first coating always contains polyethyleneglycol and the fourth coating always contains cellulose acetate phthalate (CAP)

2.  The controlled release pharmaceutical composition of claim 1, wherein:

    -   the excipient is selected from the group consisting of polyvinylpyrrolidone, lactose and dibasic calcium phosphate;
    -   the first coating comprises, further to polyethylenglycol, a mixture of ethyl cellulose, diethyl phthalate and polyethylene glycol;
    -   the second coating further comprises a mixture of at least one of the following components selected from the group consisting of methacrylic acid copolymers, methylcellulose, polyvinylpyrrolidone;
    -   the third coating comprises one or more materials selected from the group consisting of mono-, di-, and triglycerides of fatty acids, waxes, fatty alcohols and fatty acids; and
    -   the fourth coating comprises a mixture selected from the group consisting of cellulose acetate phthalate with diethyl phthalate, hydroxypropyl methyl cellulose phthalate with diethyl phthalate and cellulose acetate trimellitate with diethyl phthalate, and
    -   the liquid administration vehicle comprises suspending, sweetening, buffering, preserving and flavoring agents.

3.  The controlled release pharmaceutical composition of claim 2 wherein the second coating contains the same mixture forming the fourth coating.

4.  The controlled pharmaceutical composition of Claim 1, wherein said microgranules have naproxen contents of 80% - 90% by weight prior to coating a homogeneous and uniformly smooth surface, a substantially spherical shape, an apparent density of between 300 and 800 g/l, and a low friability.

5.  The controlled release pharmaceutical composition of Claim 2, wherein the mixture forming the first coating applied to the microgranules, contains diethyl phthalate in a range from 10% to 30% by weight compared to the weight of the total ingredients of the first coating, and containing polyethylene glycol in a range from 0.1% to 5% by weight as compared to the total weight of the first coating mixture.

6.  The controlled release pharmaceutical dosage form of Claim 2, wherein the second coating of hydrophilic material applied to the first coating contains diethyl phthalate in a range from 10% to 30% by weight as compared to the total weight of the ingredients of the second coating mixture.

7.  The controlled release pharmaceutical dosage form of Claim 2, wherein the fourth coating contains diethyl phthalate in a range from 10% to 30% by weight as compared to the total weight of the ingredients of the fourth coating mixture.

8. The controlled release pharmaceutical dosage form of claims 1 through 7 wherein said coated microgranules have dimensions of between 90 and 300 $\mu$m.

9. The controlled release pharmaceutical dosage form of Claims 1 through 8, wherein the mixture of ingredients is suspended in water to obtain a liquid dosage formulation for immediate use.

10. A pharmaceutical composition according to Claim 9, wherein the suspension of said coated microgranules in a liquid vehicle maintains the release characteristics of the microgranules essentially unchanged for at least a month.

11. A pharmaceutical composition according to Claims 1 through 10, wherein a single administration ensures naproxen plasma levels higher than 18 $\mu$g/ml for at least 24 hours.

**Patentansprüche**

1. Eine pharmazeutische Zusammensetzung mit kontrollierter Freisetzung geeignet für eine einmal tägliche Verabreichung von Naproxen in einer Flüssig-Suspension, umfassend:

   - ein Mikrogranulat, das Naproxen als Arzneistoff und mindestens einen Arzneimittelträger (Excipiens) enthält, mit einer Größe im Bereich von 50 bis 500 $\mu$m, wobei das Mikrogranulat vor der Beschichtung im wesentlichen keine Eigenschaft von kontrollierter Freisetzung besitzt,
   - eine Reihe von vier aufeinanderfolgenden Polymerlagen, die das Mikrogranulat beschichten, wobei diese Reihe besteht aus:
   - einer ersten Beschichtung, die direkt auf das Mikrogranulat aufgebracht wird und dem Mikrogranulat die Eigenschaft einer kontrollierten Freisetzung in Übereinstimmung mit einem vorherbestimmten Freisetzungsprofil verleiht,
   - einer zweiten Beschichtung mit hydrophilen Eigenschaften,
   - einer dritten Beschichtung mit lipophilen Eigenschaften, und
   - einer vierten Beschichtung mit Enteric-Beschichtungs-Eigenschaften;

   ein Träger für die flüssige Verabreichung des beschichteten Mikrogranulats, dadurch gekennzeichnet, daß die erste Beschichtung immer Polyethylenglykol enthält und die vierte Beschichtung immer Celluloseacetatphthalat enthält.

2. Die pharmazeutische Zusammensetzung mit kontrollierter Freisetzung nach Anspruch 1, wobei:

   - der Arzneimittelträger aus der Gruppe bestehend aus Polyvinylpyrrolidon, Lactose und Calciumhydrogenphosphat ausgewählt wird;
   - die erste Beschichtung neben Polyethylenglycol eine Mischung von Ethylcellulose, Diethylphthalat und Polyethylenglycol umfaßt;
   - die zweite Beschichtung darüberhinaus eine Mischung von mindestens einer der folgenden Komponenten ausgewählt aus der Gruppe bestehend aus Methacrylsäure-Copolymeren, Methylcellulose, Polyvinylpyirolidon umfaßt;
   - die dritte Beschichtung eine oder mehrere Komponenten ausgewählt aus der Gruppe bestehend aus Mono-, Di- oder Triglyceriden von Fettsäuren, Wachse, Fettalkohole und Fettsäuren umfaßt; und
   - die vierte Beschichtung eine Mischung ausgewählt aus der Gruppe bestehend aus Celluloseacetatphtalat mit Diethylphtalat, Hydroxypropylmethylcellulosephthalat mit Diethylphtalat und Celluloseacetattrimellitat mit Diethylphtalat umfaßt, und
   - der Träger für die flüssige Verabreichung Suspendiermittel, Süßungsmittel, Puffersubstanzen, Konservierungsmittel und Aromastoffe umfaßt.

3. Die pharmazeutische Zusammensetzung mit kontrollierter Freisetzung nach Anspruch 2, wobei die zweite Beschichtung die gleiche Mischung enthält, aus der die vierte Beschichtung besteht.

4. Die pharmazeutische Zusammensetzung mit kontrollierter Freisetzung nach Anspruch 1, wobei das Mikrogranulat einen Naproxen-Gehalt von 80-90 Gew.-% aufweist, vor der Beschichtung eine homogene und einheitlich glatte Oberfläche besitzt, im wesentlichen kugelförmig ist, eine Schüttdichte von 300-800 g/l aufweist und eine sehr geringe Sprödigkeit zeigt.

5. Die pharmazeutische Zusammensetzung mit kontrollierter Freisetzung nach Anspruch 2, wobei die Mischung für die erste Beschichtung des Mikrogranulats Diethylphthalat in einer Menge von 10 bis 30 Gew.-% enthält, bezogen auf das Gewicht der gesamten Bestandteile der ersten Beschichtung, und Polyethylenglycol in einer Menge von 0,1 bis 5 Gew.-%, bezogen auf das Gewicht der gesamten Bestandteile der ersten Beschichtungsmischung, enthält.

6. Die pharmazeutische Applikationsform mit kontrollierter Freisetzung nach Anspruch 2, wobei die auf die erste Beschichtung aufgebrachte hydrophile zweite Beschichtung Diethylphthalat in einer Menge von 10 bis 30 Gew.-%, bezogen auf das Gewicht der gesamten Bestandteile der zweiten Beschichtungsmischung, enthält.

7. Die pharmazeutische Applikationsform mit kontrollierter Freisetzung nach Anspruch 2, wobei die vierte Beschichtung Diethylphthalat in einer Menge von 10 bis 30 Gew.-%, bezogen auf das Gewicht der gesamten Bestandteile der vierten Beschichtungsmischung, enthält.

8. Die pharmazeutische Applikationsform mit kontrollierter Freisetzung nach den Ansprüchen 1 bis 7, wobei besagtes beschichtetes Mikrogranulat eine Größe von 90 bis 300 μm besitzt.

9. Die pharmazeutische Applikationsform mit kontrollierter Freisetzung nach den Ansprüchen 1 bis 8, wobei die Mischung der Bestandteile in Wasser suspendiert wird, um eine flüssige Dosierungs-Zusammensetzung für den sofortigen Gebrauch zu erhalten.

10. Eine pharmazeutische Zusammensetzung nach Anspruch 9, wobei die Suspension des Mikrogranulats in einem flüssigen Träger die Freisetzungs-Charakteristik des Mikrogranulats im wesentlichen für mindestens einen Monat unverändert aufrecht erhält.

11. Eine pharmazeutische Zusammensetzung nach den Ansprüchen 1 bis 10, wobei eine einmalige Verabreichung Naproxen-Plasma-Konzentrationen höher als 18 μg/ml für wenigstens 24 Stunden gewährleistet.

**Revendications**

1. Composition pharmaceutique à libération contrôlée convenable pour une administration quotidienne de naproxène dans une suspension liquide, comprenant :

- une multiplicité de microgranules contenant du naproxène en tant que composant actif et au moins un excipient, ayant des dimensions comprises dans la gamme de 50 à 500 ppm, ces microgranules n'ayant pratiquement aucune propriété de libération contrôlée avant revêtement,
- une série de quatre couches polymères successives recouvrant ces microgranules, cette série consistant en :

  - un premier revêtement appliqué directement sur les microgranules, conférant des propriétés de libération contrôlée à ces microgranules selon un profil de libération prédéterminé,
  - un deuxième revêtement appliqué doté de caractéristiques hydrophiles,
  - un troisième revêtement appliqué doté de caractéristiques lipophiles, et
  - un quatrième revêtement appliqué doté des caractéristiques d'un revêtement entérique,

- un véhicule pour l'administration sous forme liquide pour les microgranules revêtus ci-dessus,

caractérisée en ce que le premier revêtement contient toujours du polyéthylène glycol et que le quatrième revêtement contient toujours de l'acétate phtalate de cellulose (CAP).

2. Composition pharmaceutique à libération contrôlée suivant la revendication 1, dans laquelle :

- l'excipient est choisi dans le groupe consistant en polyvinyl pyrrolidone, lactose et phosphate dibasique de calcium,
- le premier revêtement comprend, en plus du polyéthylène glycol, un mélange d'éthyl cellulose, de phtalate de diéthyle et de polyéthylène glycol,
- le deuxième revêtement comprend de plus un mélange d'au moins l'un des composants suivants choisi dans le groupe consistant en copolymères d'acide méthacrylique méthylcellulose, polyvinyl pyrrolidone,
- le troisième revêtement comprend un ou plusieurs matériaux choisis dans le groupe consistant en mono-, di- et triglycérides d'acides gras, cires, alcools gras et acides gras, et

- le quatrième revêtement comprend un mélange choisi dans le groupe consistant en acétate phtalate de cellulose et phtalate de diéthyle, phtalate d'hydroxypropyl méthyl cellulose et phtalate de diéthyle, et acétate triméllitate de cellulose et phtalate de diéthyle, et
- le véhicule pour l'administration sous forme liquide comprend des agents de suspension, des édulcorants, des tampons, des agents conservateurs et des arômes.

3. Composition pharmaceutique à libération contrôlée suivant la revendication 2, dans laquelle le deuxième revêtement contient le même mélange que celui qui forme le quatrième revêtement.

4. Composition pharmaceutique à libération contrôlée suivant la revendication 1, dans laquelle ces microgranules ont des teneurs en naproxène de 80 a 90% en poids avant revêtement, une surface homogène et uniformément lisse, une forme pratiquement sphérique, une densité apparente comprise entre 300 et 800 g/l et une faible friabilité.

5. Composition pharmaceutique à libération contrôlée suivant la revendication 2, dans laquelle le mélange formant le premier revêtement appliqué sur les microgranules, contient du phtalate de diéthyle dans une gamme de 10% à 30% en poids par rapport au poids du total des composants du premier revêtement, et contient du polyéthylène glycol dans une gamme de 0,1% à 5% en poids par rapport au poids total du premier mélange de revêtement.

6. Forme d'administration pharmaceutique à libération contrôlée suivant la revendication 2, dans laquelle le deuxième revêtement de matière hydrophile appliqué sur le premier revêtement contient du phtalate de diéthyle dans une gamme de 10% à 30% en poids par rapport au poids total des composants du deuxième mélange de revêtement.

7. Forme d'administration pharmaceutique à libération contrôlée suivant la revendication 2, dans laquelle le quatrième revêtement contient du phtalate de diéthyle dans une gamme de 10% à 30% en poids par rapport au poids total des composants du quatrième mélange de revêtement.

8. Forme d'administration pharmaceutique à libération contrôlée suivant les revendications 1 à 7, dans laquelle ces microgranules revêtus ont des dimensions comprises entre 90 et 300 $\mu$m.

9. Forme d'administration pharmaceutique à libération contrôlée suivant les revendications 1 à 8, dans laquelle le mélange de composants est mis en suspension dans de l'eau pour obtenir une formulation d'administration liquide pour un usage immédiat.

10. Composition pharmaceutique suivant la revendication 9, dans laquelle la suspension de ces microgranules revêtus dans un véhicule liquide conserve les caractéristiques de libération des microgranules pratiquement intactes pendant au moins un mois.

11. Composition pharmaceutique suivant les revendications 1 à 10, dans laquelle une administration unique assure des taux de naproxène dans le plasma supérieurs à 18 $\mu$g/ml pendant au moins 24 heures.

Fig.1